# EUROPÄISCHE PATENTSCHRIFT

(11) **EP 2 508 210 B1**
(45) Veröffentlichungstag und Bekanntmachung des Hinweises auf die Patenterteilung: **03.01.2018**
(21) Anmeldenummer: 12001660.5
(22) Anmeldetag: 10.03.2012
(51) Int. Cl.: A61L 24/00

(54) **Plastisch verformbares, biodegradierbares Hämostyptikum**
Plastic mouldable biodegradable hemostyptic
Hémostyptique préformé en plastique biodégradable

(30) Priorität: 06.04.2011 DE 102011016277
(43) Veröffentlichungstag der Anmeldung: 10.10.2012
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- DE-A1-102005 002 703
- DE-B3-102004 060 666
- US-A- 5 681 873
- US-A1- 2006 002 976

## Beschreibung

Die Erfindung betrifft ein plastisch verformbares, biodegradierbares Hämostyptikum, das zur mechanischen Versiegelung von blutendem Knochengewebe einsetzbar ist, ein Verfahren zum Formen eines solchen plastisch verformbaren, biodegradierbaren Hämostyptikums und ein medizinisches Implantat, das eine Beschichtung aufweist, die ein solches plastisch verformbares, biodegradierbares Hämostyptikum umfasst.

Bei Operationen wird die Blutstillung je nach anatomischen Gegebenheiten durch unterschiedliche Verfahren, wie z.B. die Elektrokoagulation (Kauterisierung) der Blutgefäße, durchgeführt. Bei einer Reihe von Operationen im Schädelbereich und vor allem am Sternum wird bei den dort auftretenden starken Blutungen aufgrund der anatomischen Situation Knochenwachs zur Versiegelung der Kapillargefäße und damit der Blutstillung eingesetzt. Dabei wird das Knochenwachs vom Operateur zuerst mit der Hand weich geknetet und anschließend direkt auf bzw. in die blutenden Knochenareale gedrückt. Dabei kommt es zu einer Stauung des Blutflusses, wodurch Hämatome entstehen und die versorgenden Gefäße durch Fibrin letztlich verschlossen werden.

Knochenwachs ist seit dem 19. Jahrhundert bekannt und enthält im Allgemeinen gebleichtes Bienenwachs und einen Weichmacher. Als Weichmacher werden unter anderem Mandelöl, Vaseline, Palmitinsäure, Isopropylester und Myristinsäureisopropylester verwendet. Die üblichen Knochenwachse sind bei Raumtemperatur zähe und relativ schwer knetbare Massen. Der in dem Bienenwachs enthaltene Weichmacher dient dazu, dass beim Kneten des Knochenwachses das Material aufgrund der Handwärme erweicht und plastisch verformbar wird. Knochenwachse auf der Basis von Bienenwachs gelten als nicht biodegradierbar im humanen Organismus. Häufige Bestandteile des Bienenwachses sind Ester von Myristinsäure und höheren Alkoholen, wie z.B. Myristinsäuremyricylester. Es wird angenommen, dass der humane Organismus nicht über geeignete Enzyme verfügt, die die sehr hydrophen Ester in überschaubaren Zeiträumen abbauen können. Die gegenwärtig im Handel erhältlichen Knochenwachse weisen eine sehr gute hämostyptische Wirkung auf, führen andererseits jedoch nicht selten zu Schädigungen im humanen Organismus (S. E. Katz, J. Rotmann: Adverse effects of bone wax in surgery of the orbit. Ophthal Plast. Reconstr. 1996, 12 (2) 121-126; M. Lavigne et al.: Bone-wax granuloma after femoral neckosteoplasty. Can. J. Surg. 2008, 51 (3) E58-60; R. T. Allison: Foreign body reactions and an associated histological artifact due to bone wax. Br. J. Biomed. Sci. 1994, 51 (1) 14-17; O. Eser et al.: Bone wax as a cause of foreign body reaction after lumbar disc surgery: A case report. Adv. Ther. 2007, 24 (3) 594-7). Als Hauptvorteil von konventionellen Knochenwachsen kann die sehr gute Haftwirkung auf feuchtem und auch fettigem Knochengewebe betrachtet werden.

Aus dem Stand der Technik sind einige Alternativen zu herkömmlichem Knochenwachs bekannt.

Beispielsweise offenbart die EP 0109310 eine wachsartige Masse, die auf Calciumfettsäuresalzen und Oligomeren von Hydroxycarbonsäuren basiert.

Aus der US 4595713 A, DE 3229540 A1, DE 1985889 A1, EP 1142597 A1 sind wachsartige Zusammensetzungen bekannt, die Oligoester von Hydroxycarbonsäuren, wie zum Beispiel Milchsäure und 6-Hydroxycarbonsäure, enthalten. Es hat sich gezeigt, dass beim Einsatz dieser wachsartigen Zusammensetzungen während des hydrolytischen Abbaus saure Degradationsprodukte entstehen, die das Knochengewebe infolge einer lokalen pH-Wert Absenkung beeinträchtigen.

DE 10 2005 002 703 A1 offenbart eine antibiotische Beschichtung von Implantaten, die einen Matrixbildner, ein Additiv und ein Antibiotikum enthält, wobei als Matrixbildner bspw. gesättigte Glycerin-1,2,3-trifettsäureester in Betracht kommen, die zum Teil als Partikel vorliegen können.
US 2006/0002976 A1 offenbart ein absorbierbares, biokompatibles Hämostyptikum. Dieses Hämostyptikum enthält ein Carbonsäuresalz als Füllstoff und ein Dispergiermittel. Bei dem Carbonsäuresalz handelt es sich um Fettsäuresalze mit anorganischen Basen. Das biokompatible Hämostyptikum enthält ferner eine zweite Kom ponente, welche bevorzugt flüssig ist und aus einer Vielzahl von Substanzen ausgewählt ist.

US 5,681,873 A offenbart eine biodegradierbare pharmazeutische Zusammensetzung, die ein thermoplastisches Polymer auf der Basis von Poly(caprolacton) sowie ein Mittel zur Steuerung der Kristallisation enthält. Als Mittel zur Steuerung der Kristallisation werden anorganische Salze eingesetzt

Eine vielversprechende Entwicklung stellen Zusammensetzungen auf der Basis von Polyethern dar (US 2009/286886 A1, US 2011/002974 A1). Als Polyether können beispielsweise Poly(propylenglykol-co-ethylenglykole) verwendet werden. Diese Zusammensetzungen sind bei Handwärme knetbar und streichfähig. Als nachteilig ist jedoch die gute Löslichkeit dieser Polyether in wässrigem Milieu zu sehen. Dies führt dazu, dass die Haftung dieser Zusammensetzungen im Fall von stark blutendem Knochengewebe infolge der Anlösung der wachsartigen Zusammensetzung erschwert sein kann. Dadurch kann es unter Umständen zu Nachblutungen kommen, die wiederum eine schnelle Anlösung oder Auflösung der Siegelung bedingen. Besonders vorteilhaft an diesen Gemischen ist jedoch, dass sie keinerlei Barrierefunktion für die Knochenheilung besitzen und vollständig renal ausgeschieden werden (A. Suwan et al.: Controversial role of two different local haemostatic agents on bone healing. J. am Sci. 2010; 6 (12) 15-163).

Es besteht daher weiterhin Bedarf an einem plastisch verformaren, biodegradierbaren Hämostyptikum, das die vorstehend beschriebenen Nachteile nicht aufweist. Dieses Hämostyptikum soll ähnlich dem bisher handelsüblichen Knochenwachs bei Körpertemperatur eine knetbare und plastisch verformbare Masse sein. Die Zähigkeit dieses Hämostyptikums sollte so hoch sein, dass die Masse dem Blutungsdruck standhält. Ferner sollte das Hämostyptikum eine hinreichend große Kohäsion aufweisen, so dass es bei Kontakt mit Blut oder anderen wässrigen Flüssigkeiten nicht auseinanderfällt oder sich innerhalb weniger Minuten auflöst. Weiterhin sollte das Hämostyptikum keine aciden oder basischen Bestandteile in größeren Mengen abgeben, um das Knochengewebe nicht durch einen nicht physiologischen pH-Wert zu schädigen. Darüber hinaus sollte das Material biodegradierbar sein oder renal ausgeschieden werden können, damit keine dauerhafte Barrierewirkung durch das Material den Heilungsprozess des Knochengewebes behindern kann. Außerdem sollte die Masse beim Weichkneten und beim Applizieren nicht an Gummihandschuhen haften bleiben.

Erfindungsgemäß besteht daher die Aufgabe, ein vorteilhaftes plastisch verformares, biodegradierbares Hämostyptikum bereitzustellen, das zur mechanischen Versiegelung von blutendem Knochengewebe einsetzbar ist. Eine weitere erfindungsgemäße Aufgabe besteht darin, ein Verfahren zum Formen eines solchen plastisch verformaren, biodegradierbaren Hämostyptikums zur Verfügung zu stellen. Die Erfindung stellt sich ferner zur Aufgabe, ein medizinisches Implantat bereitzustellen, das eine Beschichtung aufweist, die ein solches plastisch verformares, biodegradierbares Hämostyptikum umfasst.

Diese Aufgaben werden gelöst durch die Gegenstände der unabhängigen Ansprüche.

Die Erfindung stellt demnach ein plastisch verformbares, biodegradierbares Hämostyptikum bereit, das (b) wenigstens einen zumindest teilweise in partikulärer Form vorliegenden Füllstoff, wobei die Komponente (b) aus der Gruppe ausgewählt ist, die aus Polymeren von wenigstens einem Alkylenoxid, Copolymeren von wenigstens einem Alkylenoxid und Calciumverbindungen besteht, und(c) wenigstens einen gesättigten Fettsäureester, gekennzeichnet durch(a) wenigstens einen gesättigten Glycerin-1,2,3-trifettsäureester mit einer Schmelztemperatur von mehr als 37°C, wobei die Calciumverbindungen Salze wenigstens einer anorganischen Säure sind und wobei die Komponente (b) eine Schmelztemperatur von mehr als 37°C und bei einer Temperatur von 25°C eine Löslichkeit von wenigstens 100 Gramm pro Liter Wasser aufweist und der wenigstens eine gesättigten Fettsäureester eine Schmelztemperatur von nicht mehr als 30°C und bei einer Temperatur von 25°C eine Löslichkeit von weniger als 50 Gramm pro Liter Wasser aufweist, enthält.

Die Erfindung stellt ferner zur Verfügung ein Verfahren zum Formen eines solchen plastisch verformbaren, biodegradierbaren Hämostyptikums, umfassend die Schritte: (a) Bereitstellung eines erfindungsgemäßen plastisch verformbaren, biodegradierbaren Hämostyptikums, (b) Erwärmen des plastisch verformbaren, biodegradierbaren Hämostyptikums auf eine Temperatur im Bereich von 35 - 40°C und (c) Formen des erwärmten plastisch verformbaren, biodegradierbaren Hämostyptikums. Außerdem stellt die Erfindung ein medizinisches Implantat bereit, aufweisend eine Beschichtung, die ein solches plastisch verformbares, biodegradierbares Hämostyptikum umfasst.

Die Erfindung basiert zum Teil auf der überraschenden Erkenntnis, dass durch das Zusammenwirken der Komponenten (a), (b) und (c) eine Mischung bereitgestellt werden kann, die effektiv als Hämostyptikum wirkt und zur Versiegelung von blutendem Knochengewebe eingesetzt werden kann. Besonders überraschend ist dabei, dass das erfindungsgemäße Hämostyptikum auf der Basis der Komponenten (a) bis (c) als wachsartige, knetbare Masse vorliegt, die sowohl auf trockenen als auch auf feuchten Oberflächen haftet. Die Zähigkeit und die mechanische Stabilität dieser Mischung sind überraschenderweise so hoch, dass diese als effektives Hämostyptikum für die Blutstillung verwendet werden kann und den bei Verletzungen vorherrschenden Blutungsdrücken standhält. Obgleich die Mischung biodegradierbar ist, weist sie überraschenderweise eine derart große mechanische Stabilität auf, dass sie beim Kontakt mit Blut nicht zerfällt.

Ohne an eine Theorie gebunden sein zu wollen, scheinen diese vorteilhaften Eigenschaften auf der Bildung einer stabilen Matrix zu beruhen, in der die in partikulärer
Form vorliegenden Füllstoffe miteinander über den wenigstens einen gesättigten Glycerin-1,2,3-trifettsäureester, der eine Schmelztemperatur von mehr als 37 °C aufweist, verbunden sind. Durch den weiteren Einsatz wenigstens eines gesättigten Fettsäureesters mit einer Schmelztemperatur von nicht mehr als 30°C, die bei einer

Temperatur von 25°C eine Löslichkeit von weniger als 50 Gramm pro Liter Wasser aufweist, wird gewährleistet, dass die gebildete Mischung als solche plastisch verformbar ist. Dabei wird die mechanische Stabilität des geschaffenen Hämostyptikums jedoch überraschenderweise nicht beeinträchtigt. In diesem Zusammenhang ist es insbesondere überraschend, dass die Verbindung mit einer Schmelztemperatur von nicht mehr als 30°C, die bei einer Temperatur von 25°C eine Löslichkeit von weniger als 50 Gramm pro Liter Wasser aufweist, nicht aus dem Hämostyptikum austritt oder entweicht, sondern fest in dem wohl auf der Bildung einer Matrix beruhenden System eingeschlossen bleibt.

Erfindungsgemäß wird ein Hämostyptikum zur Verfügung gestellt. Unter einem Hämostyptikum werden erfindungsgemäß Zusammensetzungen verstanden, die blutstillende Eigenschaften aufweisen.

Das erfindungsgemäße Hämostyptikum ist plastisch verformbar. Unter plastischer Verformbarkeit wird vorliegend die Fähigkeit des Hämostyptikums verstanden, sich unter einer Krafteinwirkung irreversibel zu verformen und diese Form nach der Krafteinwirkung beizubehalten.

Das erfindungsgemäße Hämostyptikum ist ferner biodegradierbar. Als biodegradierbar werden vorliegend Stoffe bezeichnet, die vom humanen Organismus abgebaut werden können.

Das Hämostyptikum des vorliegenden Erfindung weist in Wasser bei einer Temperatur von 25 °C vorzugsweise einen pH-Wert im Bereich von 5,0 bis 9,0, mehr bevorzugt einen pH-Wert im Bereich von 5,5 bis 8,5 noch mehr bevorzugt einen pH-Wert im Bereich von 6,0 bis 8,0 und besonders bevorzugt einen pH-Wert im Bereich von 6,5 bis 7,5 auf.

Gemäß einer bevorzugten Ausführungsform enthält das erfindungsgemäße Hämostyptikum keine Oligoester von Hydroxycarbonsäuren und insbesondere keine Oligoester von Milchsäure oder 6-Hydroxycapronsäure.

Das erfindungsgemäße biodegradierbare Hämostyptikum enthält als Komponente (a) wenigstens einen gesättigten Glycerin-1,2,3-trifettsäureester mit einer Schmelztemperatur von mehr als 37 °C.

Unter gesättigtem Glycerin-1,2,3-trifettsäureester wird gemäß dem allgemeinen Sprachgebrauch ein Glycerinester verstanden, der drei Fettsäurereste aufweist, die jeweils frei von Kohlenstoff-Kohlenstoff-Mehrfachbindungen sind.

Die Schmelztemperatur des als Komponente (a) eingesetzten wenigsten einen gesättigten Glycerin-1,2,3-trifettsäureesters beträgt mehr als 37°C, vorzugsweise mehr als 40°C, mehr bevorzugt mehr als 42°C und noch mehr bevorzugt mehr als 45°C.

Im Rahmen der gesamten vorliegenden Erfindung bezeichnet die Schmelztemperatur die Temperatur, bei der der betreffende Stoff vom festen in den flüssigen Aggregatzustand übergeht. Vollzieht sich bei einem Stoff der Übergang vom festen in den flüssigen Aggregatzustand nicht bei einer bestimmten Temperatur, sondern in einem Schmelzbereich, so wird im Rahmen der Erfindung unter Schmelztemperatur die tiefere der beiden Grenztemperaturen dieses Schmelzbereichs verstanden.

Durch eine Schmelztemperatur in dem angegebenen Bereich wird gewährleistet, dass bei der Körpertemperatur von 37 °C der gesättigte Glycerin-1,2,3,-trifettsäureester nicht aufschmilzt und das Hämostyptikum nicht erweicht.

Die wenigstens eine Komponente (a) ist aus der Gruppe ausgewählt, die aus gesättigten Glycerin-1,2,3-trifettsäureestern besteht, die wenigstens einen Fettsäurerest mit 12 - 28 Kohlenstoffatomen, mehr bevorzugt 14 - 24 Kohlenstoffatomen und noch mehr bevorzugt 12 - 22 Kohlenstoffatomen aufweisen. Gemäß einer besonders bevorzugten Ausführungsform ist die Komponente (a) aus der Gruppe ausgewählt, die aus gesättigten Glycerin-1,2,3-trifettsäureestern besteht, die drei Fettsäurereste mit 12 - 28 Kohlenstoffatomen, vorzugsweise 14 - 24 Kohlenstoffatomen und noch mehr bevorzugt 12 - 22 Kohlenstoffatomen aufweisen. Die Fettsäurereste können verzweigt sein, sind aber vorzugsweise unverzweigt. Ferner können die Fettsäurereste gegebenenfalls substituiert sein. Vorzugsweise sind die Fettsäurereste jedoch nicht substituiert. Die Fettsäurereste sind vorzugsweise aus der Gruppe ausgewählt, die aus Fettsäureresten von Laurinsäure, Myristinsäure, Palmitinsäure, Margarinsäure, Sterinsäure, Arachinsäure und Behensäure besteht.

Die als Komponente (a) eingesetzten Ester können identische Reste aufweisen. Andererseits kann es sich bei der Komponente (a) auch um einen Mischester handeln. Unter Mischester sind vorliegend Ester zu verstehen, die wenigstens zwei unterschiedliche Fettsäurereste aufweisen. In den Mischestern können insbesondere auch drei unterschiedliche Fettsäurereste vorhanden sein.

Gemäß einer bevorzugten Ausführungsform ist die Komponente (a) aus der Gruppe ausgewählt, die aus Glycerin-1,2,3-behensäureester, Glycerin-1,2,3-tristearinsäureester und Glycerin-1,2,3-tripalmitinsäureester besteht.

Die Komponente (a) fungiert in dem erfindungsgemäßen Hämostyptikum offenbar als Matrixbildner. Sie scheint dabei die Rolle eines Bindemittels auszuüben, das für eine außergewöhnlich hohe mechanisch Stabilität des Hämostyptikums durch eine starke Kohäsion mit den übrigen Komponenten, insbesondere dem als Komponente (b) vorliegenden Füllstoff, sorgt.

Bei der wenigstens einen Komponente (b) handelt es sich um wenigstens einen zumindest teilweise in partikulärer Form vorliegenden Füllstoff mit einer Schmelztemperatur von mehr als 37°C. Dabei ist die Komponente (b) aus der Gruppe ausgewählt, die aus Polymeren von wenigstens einem Alkylenoxid, Copolymeren von wenigstens einem Alkylenoxid und Calciumverbindungen besteht, wobei die Calciumverbindungen Salze wenigstens einer anorganischen Säure sind

Die Schmelztemperatur des als Komponente (b) eingesetzten Füllstoffs beträgt vorzugsweise mehr als 40°C, mehr bevorzugt mehr als 42°C und noch mehr bevorzugt mehr als 45°C. Dadurch wird gewährleistet, dass bei der Körpertemperatur von 37 °C der Füllstoff nicht aufschmilzt und das Hämostyptikum nicht erweicht.

Der als Komponente (b) eingesetzte Füllstoff ist vorzugsweise hydrophil. Der Füllstoff weist bei einer Temperatur von 25 °C eine Löslichkeit von wenigsten 100 Gramm pro Liter Wasser und noch mehr bevorzugt eine Löslichkeit von wenigsten 130 Gramm pro Liter Wasser auf.

Der Füllstoff liegt wenigstens zumindest teilweise in partikulärer Form vor.

Vorzugsweise liegen wenigstens 30 Prozent, mehr bevorzugt wenigstens 50 Prozent, noch mehr bevorzugt wenigstens 70 Prozent, besonders bevorzugt wenigstens 90 Prozent, ganz besonders bevorzugt wenigstens 95 Prozent und insbesondere wenigstens 99 Prozent des Füllstoffs in partikulärer Form vor.

Die Partikel des Füllstoffs können in unterschiedlichen Geometrien vorliegen. Beispielsweise können die Partikel des Füllstoffs eine sphärische Gestalt, eine kubische Gestalt oder aber eine unregelmäßige Form aufweisen.

Die Partikel des Füllstoffs weisen vorzugsweise einen mittleren Teilchendurchmesser im Bereich von 50 nm bis 500 µm, mehr bevorzugt im Bereich von 100 nm bis 100 µm und noch mehr bevorzugt im Bereich von 500 nm bis 100 µm auf. Unter mittlerem Teilchendurchmesser wird vorliegend verstanden, dass wenigstens 50 % der Teilchen den angegebenen Teilchendurchmesser aufweisen.

Gemäß einer bevorzugten Ausführungsform ist die Komponente (b) ein hydrophiler Füllstoff. Als besonders vorteilhaft haben sich als Komponente (b) Füllstoffe erwiesen, die in Gegenwart von Wasser einen pH-Wer im Bereich von 6 - 8 aufweisen.

Gemäß einer Ausführungsform der Erfindung ist der als Komponente (b) geeignete Polyether aus der Gruppe ausgewählt, die aus Polymeren von wenigsten Ethylenoxid, Polymeren von wenigsten Propylenoxid, Copolymeren von wenigstens Ethylenoxid, Copolymeren von wenigsten Propylenoxid und Copolymeren von wenigsten Ethylenoxid und Propylenoxid besteht. Bei den Copolymeren von Ethylenoxid und Propylenoxid kann es sich auch um Blockcopolymere, insbesondere Poloxamere, handeln.

Als besonders vorteilhaft hat sich dabei die Verwendung von Polyalkylethern, wie zum Beispiel Poloxameren, Polyethylenglykolen oder Poly(propylenglykol-co-ethylenglykol), erwiesen.

Gemäß einer weiteren bevorzugten Ausführungsform ist der als Komponente (b) verwendete Polyether aus der Gruppe ausgewählt, die aus Polyethylenglykol 35.000, Polyethylenoxid 100.000, Polyethylenoxid 300.000, Polyethylenoxid 1.000.000 und Poly(propylenglykol-co-ethylenglycol) mit einem Polyoxyethylengehalt im Bereich von 50 - 80 Gewichtsprozent besteht.

Ferner kann es bevorzugt sein, wenn als Komponente (b) ein hydrophiler Füllstoff eingesetzt wird, der eine Erweichungstemperatur von mehr als 45°C aufweist. Es hat sich herausgestellt, dass hydrophile Füllstoffe mit einem Erweichungspunkt von 45°C eine überraschend große mechanische Stabilität des erfindungsgemäßen Hämostyptikums bewirken. Erfindungsgemäß handelt es sich bei der Komponente (b) um eine Calciumverbindung, die das Salz wenigstens einer anorganischen Säure ist.

Unter Calciumsalz wird erfindungsgemäß ein Salz verstanden, das als kationische Komponente wenigsten Calciumionen aufweist. Neben den Calciumionen können in dem Salz weitere kationische Komponenten vorliegen. Bei diesen weiteren kationischen Komponenten kann es sich beispielsweise um Kationen von Elementen der ersten oder zweiten Hauptgruppe des Periodensystems der Elemente, insbesondere von Natrium, Magnesium oder Strontium, handeln. Neben Anionen, die sich aus einer anorganischen Säure ableiten, können in der Calciumverbindung weitere anionische Komponenten enthalten sein. Bei diesen weiteren anionischen Komponenten kann es sich beispielsweise um Halogenidanionen oder Hydroxidanionen handeln. Vorzugsweise ist das Salz wenigstens einer anorganischen Säure aus der Gruppe ausgewählt, die aus Calciumsalzen von Kohlensäure, Phosphorsäuren und Schwefelsäure besteht. Demnach kann es sich bei dem Calciumsalz zum Beispiel um Calci

umcarbonat, Dolomit, α-Tricalciumcarbonat, β-Tricalciumcarbonat, Hydroxylapatit, Carbonatapatit, Octacalciumphosphat, amorphisiertes Calciumphosphat, β-Tricalciumsulfat, Calciumsulfat-Dihydrat und Calciumsulfat-Hemihydrat handeln. Diese Calciumsalze sind sehr gut biokompatibel und werden durch Einwirkung von Osteoklasten oder auch durch einfaches Auflösen abgebaut. Als besonders vorteilhaft hat sich der Einsatz von Calciumsulfat-Hemihydrat herausgestellt. Ein erfindungsgemäßes Hämostyptikum, das Calciumsulfat-Hemihydrat als Komponente (b) enthält, kann überraschenderweise durch Einwirkung von Wasser oder wässrigen Flüssigkeiten, wie Blut, selbständig aushärten, ohne dass dabei die außerordentlich hohe Zähigkeit und mechanische Stabilität des Hämostyptikums verloren geht.

Es hat sich herausgestellt, dass die Komponente (b) in dem erfindungsgemäßen Hämostyptikum für eine starke Kohäsion mit den übrigen Komponenten, insbesondere dem als Komponente (a) eingesetzten gesättigten Glycerin-1,2,3-trifettsäureester mit einer Schmelztemperatur von mehr als 37°C sorgt. Überraschenderweise scheint die Komponente (b) ferner als Volumenbildner zu wirken. So wurde festgestellt, dass die Komponente (b) nach längerem Kontakt mit Wasser oder wässrigen Flüssigkeiten, wie zum Beispiel Blut, aus dem erfindungsgemäßen Hämostyptikum herausgelöst werden kann, ohne dass jedoch die mechanische Stabilität des Hämostyptikums beeinträchtigt wird. Durch das Herauslösen werden in dem Hämostyptikum überraschenderweise Hohlräume und in der Folge ein Porensystem geschaffen, das es zum Beispiel Knochengewebe erlaubt, das Hämostyptikum zu penetrieren, wodurch der Heilungsprozess gegenüber Hämostyptika, die eine Barrierewirkung entfalten, deutlich beschleunigt werden kann.

Das Hämostyptikum der vorliegenden Erfindung enthält als Komponente (c) wenigstens eine Verbindung mit einer Schmelztemperatur von nicht mehr als 30°C, die bei einer Temperatur von 25°C eine Löslichkeit von weniger als 50 Gramm pro Liter Wasser aufweist.

Gemäß einer weiteren bevorzugten Ausführungsform beträgt die Löslichkeit von Komponente (c) bei einer Temperatur von 25°C weniger als 40 Gramm pro Liter Wasser, mehr bevorzugt weniger als 30 Gramm pro Liter Wasser, noch mehr bevorzugt weniger als 25 Gramm pro Liter Wasser, besonders bevorzugt weniger als 15 Gramm pro Liter Wasser, ganz besonders bevorzugt weniger als 10 Gramm pro Liter Wasser und insbesondere weniger als 5 Gramm pro Liter Wasser.

Unter einem gesättigten Fettsäureester wird gemäß dem allgemeinen Sprachgebrauch eine Esterverbindung verstanden, die einen oder mehrere Fettsäurereste aufweist, wobei die Fettsäurereste jeweils frei von Kohlenstoff-Kohlenstoff-Mehrfachbindungen sind.

Die Fettsäurereste der als Komponente (c) verwendeten gesättigten Fettsäureester weisen vorzugsweise 8 - 28 Kohlenstoffatome, mehr bevorzugt 8 - 22 Kohlenstoffatome und noch mehr bevorzugt 8 - 18 Kohlenstoffatome auf. Diese Fettsäurereste können verzweigt sein, sind aber vorzugsweise unverzweigt. Ferner können diese Fettsäurereste Substituenten aufweisen, sie sind jedoch vorzugsweise nicht substituiert. Als Fettsäurereste kommen beispielsweise Caprylsäurereste, Pelargonsäurereste, Caprinsäurereste, Laurinsäurereste, Myristinsäurereste, Palmitinsäurereste, Margarinsäurereste, Stearinsäurereste, Arachinsäurereste und Behinsäurereste in Betracht.

Gemäß einer bevorzugten Ausführungsform ist der gesättigte Fettsäureester aus der Gruppe ausgewählt, die aus (i) Estern von mehrwertigen Alkoholen mit wenigstens einer Fettsäure, (ii) Alkylfettsäureestern und (iii) Estern von Polyethern und Fettsäuren besteht.

Gemäß einer Ausführungsform der Erfindung handelt es sich bei dem gesättigten Fettsäureester um einen Ester eines mehrwertigen Alkohols mit wenigstens einer Fettsäure. Dabei kann es sich beispielsweise um den Ester eines mehrwertigen Alkohols mit einer Kettenlänge von 2 - 4 Kohlenstoffatomen und vorzugsweise um den Ester eines mehrwertigen Alkohols mit einer Kettenlänge von 3 Kohlenstoffatomen handeln. Dieser mehrwertige Alkohol kann substituiert oder nicht substituiert sein. Als besonders bevorzugt haben sich Ester von Glycerin, 1,2-Propandiol und 1,3-Propandiol herausgestellt. Bevorzugte Ester von mehrwertigen Alkoholen mit wenigstens einer Fettsäure sind aus der Gruppe ausgewählt, die aus Monoestern von Glycerin, Diestern von Glycerin, Triestern von Glycerin, Monoestern von 1,2-Propandiol, Diestern von 1,2-Propandiol, Monoestern von 1,3-Propandiol und Diestern von 1,3-Propandiol besteht. Die Fettsäurereste der Ester von mehrwertigen Alkoholen mit wenigstens einer Fettsäure können identisch sein. Andererseits können diese Ester auch Mischester von mehrwertigen Alkoholen mit mehr als einer Fettsäure sein. Als Beispiele für Ester von mehrwertigen Alkoholen mit wenigstens einer Fettsäure können Glycerin-1,2,3-trioctylester, Mischester von Glycerin mit Caprylsäure und Laurinsäure, Propan-1,2-dioldifettsäureester und Fettsäureester von 1,3-Dihydroxy-2,2-di(hydroxymethyl)propan genannt werden.

Gemäß einer alternativen Ausführungsform kann es sich bei dem gesättigten Fettsäureester auch um einen Alkylfettsäureester handeln. Der Alkylrest der Alkylfettsäureester weist vorzugsweise eine Kettenlänge von 1 - 8 Kohlenstoffatomen, mehr bevorzugt eine Kettenlänge von 1 - 6 Kohlenstoffatomen, noch mehr bevorzugt eine Kettenlänge von 1 - 4 Kohlenstoffatomen und besonders bevorzugt eine Kettenlänge von 1 - 3 Kohlenstoffatomen auf. Der Alkylrest der Alkylfettsäureester kann verzweigt oder unverzweigt sein. Ferner kann der Alkylrest der Alkylfettsäureester gegebenenfalls substituiert sein. Dieser Alkylfettsäureester ist vorzugsweise aus der Gruppe ausgewählt, die aus Methylfettsäureestern, Ethylfettsäureestern, Propylfettsäureestern und Isopropylfettsäureestern besteht. Vorzugsweise sind die Alkylfettsäureester aus der Gruppe ausgewählt, die aus Laurinsäureethylester, Myristinsäuremethylester, Myristinsäureethylester, Myristinsäureisopropylester, Palmitinsäuremethylester, Palmitinsäureethylester und Palmitinsäureisopropylester besteht.

Gemäß einer weiteren alternativen Ausführungsform handelt es sich bei dem gesättigten Fettsäureester, der als Komponente (c) eingesetzt wird, um den Ester eines Polyethers und einer Fettsäure. Dieser Ester ist vorzugsweise aus der Gruppe ausgewählt, die aus Estern von Fettsäuren mit Polymeren von wenigstens Ethylenoxid, Estern von Fettsäuren mit Polymeren von wenigstens Propylenoxid, Estern von Fettsäuren mit Copolymeren von wenigstens Ethylenoxid und Estern von Fettsäuren mit Copolymeren von wenigstens Propylenoxid besteht. Als besonders vorteilhaft hat es sich erwiesen, dass die als Komponente (c) eingesetzten Ester von Polyethern und Fettsäuren aus der Gruppe ausgewählt sind, die aus Ethylenglykolfettsäureestern besteht.

Die Gegenwart der Komponente (c) erniedrigt in dem erfindungsgemäßen Hämostyptikum den Schmelzbereich und gewährleistet so die plastische Verformbarkeit des Hämostyptikums. Ferner wirkt die Komponente (c) als Schmiermittel, indem es die auf die übrigen Komponenten zurückzuführende Reibung deutlich herabsetzt. Darüber hinaus wurde überraschend festgestellt, dass die Komponente (c) in der Lage ist, eine ansonsten nicht zu vermeidende Rekristallisation des als Komponente (a) eingesetzten Glycerin-1,2,3-trifettsäureesters in dem Hämostyptikum zu verringern.

Die Zusammensetzung des erfindungsgemäßen Hämostyptikums mit den Komponenten (a), (b) und (c) ist nicht besonders eingeschränkt.

Gemäß einer bevorzugten Ausführungsform der Erfindung enthält das Hämostyptikum 3 - 50 Gewichtsprozent der wenigstens einen Komponente (a), 10 - 80 Gewichtsprozent der wenigstens einen Komponente (b) und 10 - 50 Gewichtsprozent der wenigstens einen Komponente (c), bezogen auf das Gesamtgewicht des Hämostyptikums.

Gemäß einer besonders besonders bevorzugten Ausführungsform enthält das Hämostyptikum der vorliegenden Erfindung 5 - 40 Gewichtsprozent der wenigstens einen Komponente (a), 20 - 75 Gewichtsprozent der wenigstens einen Komponente (b) und 20 - 40 Gewichtsprozent der wenigstens einen Komponente (c), bezogen auf das Gesamtgewicht des Hämostyptikums.

Als besonders vorteilhaft im Hinblick auf die Bildung einer stabilen Matrix hat es sich herausgestellt, dass die Komponente (b) in der Komponente (c) nur eine geringe Löslichkeit aufweist. Demnach liegt die Löslichkeit der Komponente (b) bei einer Temperatur von 25°C vorzugsweise bei weniger als 50 Gramm pro Liter an Komponente (c), mehr bevorzugt bei weniger als 20 Gramm pro Liter an Komponente (c), noch mehr bevorzugt bei weniger als 10 Gramm pro Liter an Komponente (c), besonders bevorzugt bei weniger als 5 Gramm pro Liter an Komponente (c) und ganz besonders bevorzugt bei weniger als 3 Gramm pro Liter an Komponente (c).

Zur Ausbildung einer stabilen Matrix hat es sich ferner als besonders vorteilhaft herausgestellt, dass die Komponente (b) in den Komponenten (a) und (c) des Hämostyptikums nur eine geringe Löslichkeit aufweist. Demnach liegt die Löslichkeit der wenigstens einen Komponente (b) bei einer Temperatur von 25°C vorzugsweise bei weniger als 5 Gewichtsprozent, mehr bevorzugt bei weniger als 3 Gewichtsprozent und noch mehr bevorzugt bei weniger als 1 Gewichtsprozent, bezogen auf das Gesamtgewicht der Komponenten (a) und (c).

Das Hämostyptikum der vorliegenden Erfindung kann neben den Komponenten (a), (b) und (c) gegebenenfalls weitere Bestandteile aufweisen.

Beispielsweise kann in dem erfindungsgemäßen Hämostyptikum wenigstens ein weiteres Calciumsalz enthalten sein. Dieses Calciumsalz ist vorzugsweise aus der Gruppe ausgewählt, die aus Calciumchlorid, Calciumacetat und Calciumlactat besteht. Es hat sich herausgestellt, dass die in diesen Calciumsalzen enthaltenen Calciumionen als Blutgerinnungsfaktor IV auf die sekundäre Blutgerinnung einwirken und dadurch die Blutgerinnung fördern. Der Anteil des weiteren Calciumsalzes beträgt vorzugsweise weniger als 2 Gewichtsprozent, bezogen auf das Gesamtgewicht des Hämostyptikums. Durch den Zusatz von wenigstens einem leicht löslichen Calciumsalz, das aus der Gruppe ausgewählt ist, die aus Calciumchlorid, Calciumacetat und Calciumlaktat besteht, oder dem wenigstens einen Fibrinolyseinhibitor kann erreicht werden, dass durch das erfindungsgemäße Hämostyptikum nicht nur eine mechanische Blutstillung, sondern darüber hinaus auch eine Blutstillung auf biochemischen Wege bewirkt wird.

Gemäß einer weiteren bevorzugten Ausführungsform enthält das erfindungsgemäße Hämostyptikum ferner wenigstens einen Fibrinolyseinhibitor. Als Fibrinolyseinhibitor kommen insbesondere Mitglieder aus der Gruppe der ε-Aminocarbonsäuren in Betracht. ε-Aminocarbonsäuren stellen Analoga des Lysins dar und wirken auf das Plasminogen als Vorstufe des Plasmins ein, um so die Bildung von Plasmin, das normalerweise eine enzymatische Spaltung von Fibrin bewirkt, zu behindern. Dies führt in der Folge zu einer Stabilisierung des Fibrins, wodurch die Blutgerinnung lokal verstärkt wird. Als besonders vorteilhaft für diesen Zweck haben sich die ε-Aminocarbonsäuren 6-Aminocapronsäure, 4-(Aminomethyl)benzoesäure und *trans-*4-(Aminomethyl)cyclohexan-1-carbonsäure erwiesen.

Gemäß einer weiteren bevorzugten Ausführungsform enthält das erfindungsgemäße Hämostyptikum wenigstens eine Substanz, die aus der Gruppe ausgewählt ist, die aus Antibiotika und Antiseptika besteht. Als Antibiotika sind Mitglieder aus den Gruppen der Aminoglykosid-Antibiotika, Glykopeptid-Antibiotika, Lincosamid-Antibiotika und Peptid-Antibiotika bevorzugt. Als Antiseptika kommen beispielsweise Polyhexanid, Octenidinhydrochlorid und Chlorhexidin in Betracht. Durch die Gegenwart dieser Antibiotika und/oder Antiseptika kann ein wirksamer lokaler Infektionsschutz durch das Hämostyptikum erreicht werden.

Das erfindungsgemäße Hämostyptikum kann auf unterschiedliche Weisen hergestellt werden.

Gemäß einer Ausführungsform der Erfindung wird das Hämostyptikum hergestellt, indem zunächst alle Komponenten des Hämostyptikums in ein geeignetes Mischgefäß eingebracht werden. Anschließend kann ein Vermischen der einzelnen Komponenten, beispielsweise durch Rühren, erfolgen.

Danach wird die erhaltene Mischung vorzugsweise erwärmt. Als vorteilhaft hat es sich erwiesen, die Mischung auf eine Temperatur zu erwärmen, die über dem Schmelzpunkt der Komponenten (a), (b) und (c) liegt. Beispielsweise kann das Erwärmen auf eine Temperatur von über 50°C, vorzugsweise auf eine Temperatur von über 60°C und noch mehr bevorzugt auf eine Temperatur von über 70°C erfolgen. Dabei kann die Mischung zum Beispiel auf eine Temperatur im Bereich von 50°C-100°C, vorzugsweise auf eine Temperatur im Bereich von 60°C - 95°C und noch mehr bevorzugt auf eine Temperatur im Bereich von 70°C - 90°C erwärmt werden. Erfindungsgemäß kann es demnach vorteilhaft sein, dass beim Erwärmen der Mischung eine Schmelze gebildet wird.

Das Erwärmen kann vorzugsweise für eine Zeitdauer von wenigstens 5 Minuten erfolgen. Zum Beispiel kann die Mischung für eine Zeitdauer von 5 Minuten - 4 Stunden, vorzugsweise für eine Zeitdauer von 15 Minuten - 2 Stunden und noch mehr bevorzugt für eine Zeitdauer von 30 Minuten - 60 Minuten erwärmt werden. Dabei wird die Mischung vorzugsweise gerührt. Durch das Rühren der Schmelze kann eine einfache Homogenisierung der Mischung erreicht und die Ausbildung einer Matrix durch die Komponenten (a), (b) und (c) vereinfacht werden.

Anschließend kann die erwärmte Mischung abgekühlt werden. Das Abkühlen der Mischung erfolgt vorzugsweise auf eine Temperatur im Bereich von 15°C - 30°C und mehr bevorzugt auf eine Temperatur im Bereich von 20°C - 25°C.

Danach kann die abgekühlte Mischung, falls erforderlich, weiter homogenisiert werden. Das Homogenisieren kann zum Beispiel durch ein Kneten oder Verreiben der Mischung erfolgen.

Andere Komponenten als die Komponenten (a), (b) und (c) können in die Mischung vor den Komponenten (a), (b) oder (c), gleichzeitig mit den Komponenten (a), (b) und (c) oder getrennt hiervor in einem oder mehreren der weiteren Verfahrensschritte eingebracht werden.

Gemäß einer alternativen Ausführungsform der Erfindung werden zunächst nicht sämtliche Komponenten (a), (b) und (c) in ein geeignetes Mischgefäß eingebracht, sondern nur eine oder zwei dieser Komponenten. Welche dieser Komponenten zunächst in das Mischgefäß eingebracht werden, ist üblicherweise nicht kritisch. Wird zunächst eine dieser Komponenten in das Mischgefäß eingebracht, so handelt es sich bei dieser Komponente vorzugsweise um Komponente (a) oder Komponente (c). Werden zwei dieser Komponenten zunächst in das Mischgefäß eingebracht, so handelt es sich vorzugsweise um die Komponenten (a) und (c). Die übrige Komponente oder die übrigen Komponenten, die zunächst nicht in das Mischgefäß eingebracht werden, können zu einem späteren Zeitpunkt zugegeben werden. Beispielsweise kann die Zugabe dieser Komponente(n) nach der Erwärmung oder der Abkühlung der in dem Mischgefäß bereits vorhandenen Komponente(n) erfolgen.

Das vorstehend beschriebene Hämostyptikum der vorliegenden Erfindung ist so beschaffen, dass es bei einer Temperatur von 37 °C plastisch verformbar und leicht knetbar ist und auf trockenen und feuchten Oberflächen stark haftet.

Die Erfindung betrifft daher auch ein Verfahren zum Formen des erfindungsgemä-ßen Hämostyptikums.

Bei diesem Verfahren wird zunächst ein Hämostyptikum, wie es vorstehend beschrieben ist, bereitgestellt.

Die Form der Bereitstellung des Hämostyptikums ist nicht weiter eingeschränkt. Üblicherweise wird das Hämostyptikum jedoch in einem Behältnis bereitgestellt. Dieses Behältnis ist vorzugsweise so beschaffen, das es von einem Anwender einfach geöffnet werden kann. Als geeignete Behältnisse kommen beispielsweise Dosen, Flaschen, Beutel oder Kartuschen, die jeweils mit geeigneten Verschlüssen versehen sein können, in Betracht. Das Hämostyptikum wird üblicherweise von dem Anwender nach dem Öffnen des Behältnisses aus diesem entnommen.

Nach der Bereitstellung wird das Hämostyptikum erwärmt.

Das Erwärmen erfolgt vorzugsweise auf eine Temperatur im Bereich von 35 - 40°C. Bei dieser Temperatur ist das Hämostyptikum leicht formbar.

Gemäß einer bevorzugten Ausführungsform erfolgt das Erwärmen durch wenigstens eine der Hände eines Anwenders. Das Erwärmen des erfindungsgemäßen Hämostyptikums durch den Anwender kann beispielsweise durch ein Kneten des Hämostyptikums erfolgen. Da das Hämostyptikum der vorliegenden Erfindung nicht an den Handschuhen des Anwenders haftet, ist es dem Anwender möglich, beim Erwärmen des Hämostyptikums durch wenigstens eine seiner Hände Handschuhe zu tragen. Dabei sind die Handschuhe vorzugsweise jedoch so auszuwählen, dass die Handwärme des Anwenders über den Handschuh auf das Hämostyptikum geleitet werden kann. Handschuhe aus Materialien, die entsprechend geeignete Wärmeleitfähigkeiten aufweisen, sind aus dem Stand der Technik gut bekannt und werden üblicherweise als für eine Labortätigkeit oder medizinische Tätigkeit geeignete Handschuhe vertrieben.

Gemäß einer alternativen Ausführungsform ist es ebenfalls möglich, dass Hämostyptikum auf andere Weise, vorzugsweise durch externe Wärmezufuhr, wie zum Beispiel durch Bestrahlen, zu erwärmen. Nach dieser Ausführungsform erfolgt das Erwärmen demnach nicht durch eine der Hände des Anwenders.

Als besonders bevorzugt hat sich jedoch das Erwärmen durch wenigstens eine der Hände eines Anwenders erwiesen.

Das Hämostyptikum der vorliegenden Erfindung wird ferner geformt.

Unter Formen wird vorzugsweise jede Änderung der Form oder der Geometrie des bereitgestellten Hämostyptikums verstanden.

Das Formen erfolgt vorzugsweise durch wenigstens eine der Hände eines Anwenders. Auch das Formen kann vom Anwender durchgeführt werden, während dieser Handschuhe trägt. Ein Formen des erfindungsgemäßen Hämostyptikums kann bereits zum Erwärmen des Hämostyptikums, beispielsweise durch ein Kneten des Hämostyptikums durch einen Anwender, erfolgen. Dementsprechend liegt es im Rahmen der Erfindung, dass der Schritt des Erwärmens des Hämostyptikums und der Schritt des Formens des Hämostyptikums durch eine zusammenhängende Aktion des Anwenders erfolgt. Darüber hinaus oder stattdessen kann das Formen nach dem Erwärmen des Hämostyptikums auf eine Temperatur im Bereich von 35-40°C. Das Formen kann beispielsweise dazu dienen, das Hämostyptikum in eine Form zu bringen, die für eine mechanische Versiegelung von blutendem Knochengewebe bei einer anschließenden Operation geeignet ist.

Nach dem Formen kann das Hämostyptikum von dem Anwender unmittelbar bei einer Operation eingesetzt werden.

Das Hämostyptikum der vorliegenden Erfindung kann für unterschiedliche medizinische Zwecke verwendet werden.

Gemäß einer Ausführungsform wird das Hämostyptikum zum mechanischen Verschluss blutender Knochenwunden eingesetzt. Zu diesem Zweck dient das Hämostyptikum als Knochensiegel. Es kann hierzu von einem Anwender auf oder in blutende Knochenareale gedrückt werden.

Darüber hinaus ist es auch möglich, das Hämostyptikum der Erfindung als Knochenersatzmaterial zu verwenden. Dabei kann es insbesondere von Vorteil sein, Teile von beschädigten Knochen mit dem erfindungsgemäßen Hämostyptikum zu ersetzen.

Die Erfindung betrifft auch ein medizinisches Implantat, das eine Beschichtung aufweist, die das erfindungsgemäße Hämostyptikum umfasst.

Das erfindungsgemäße Hämostyptikum kann demnach zur Beschichtung von medizinischen Implantaten verwendet werden.

Unter medizinischen Implantaten sind dabei Materialien und Vorrichtungen zu verstehen, die im Zuge eines chirurgischen Eingriffes mindestens teilweise ins Körperinnere eingebracht werden.

Die medizinischen Implantate können beispielsweise aus Metall oder aus Kunststoff gefertigt sein. Bei den medizinischen Implantaten handelt es sich vorzugsweise um Gelenkendoprothesen, Osteosynthesematerialien, Gefäßprothesen oder Herniennetze. Als Gelenkendoprothesen kommen z.B. Kniegelenkendoprothesen und Hüftgelenkendoprothesen in Betracht. Als Osteosynthesematerialien können z.B. Platten, Marknägel und Schrauben dienen.

Unter Beschichtung eines medizinischen Implantats wird erfindungsgemäß eine Schicht verstanden, die wenigstens einen Teil wenigstens einer Oberfläche des medizinischen Implantats bedeckt und auf dieser haftet.

Die Beschichtung des medizinischen Implantats umfasst das erfindungsgemäße Hämostyptikum. Demnach kann vorgesehen sein, dass die Beschichtung vollständig durch das erfindungsgemäße Hämostyptikum gebildet ist oder gegebenenfalls noch weitere Bestandteile in der Beschichtung enthalten sind.

Zur Beschichtung wird vorzugsweise das entsprechend erwärmte und geformte Hämostyptikum der vorliegenden Erfindung zumindest teilweise auf wenigstens eine Oberfläche des medizinischen Implantats aufgebracht. Dieses Aufbringen kann beispielsweise dadurch erfolgen, dass wenigstens ein Teil des erwärmten und geformten Hämostyptikums auf die wenigstens eine Oberfläche des medizinischen Implantats aufgedrückt wird oder wenigstens ein Teil des Hämostyptikums durch eine Relativbewegung des Hämostyptikums zur zu beschichtenden Oberfläche des medizinischen Implantats, bei der das Hämostyptikum mit der zu beschichtenden Oberfläche des medizinischen Implantats in Kontakt ist (zum Beispiel durch Aufstreichen), aufgetragen wird.

Die Erfindung wird durch die nachfolgenden Beispiele erläutert, die jedoch nicht als einschränkend zu verstehen sind.

### BEISPIELE:

Für die nachfolgend beschriebenen Beispiele wurden folgende Chemikalien eingesetzt:
Glycerin-1,2,3-tristearat,
Glycerin-1,2,3-tripalmitat,
Glycerin-1,2,3-trimyristat,
Glycerin-1,2,3-tristearat,
Glycerin-1,2,3-triarachinat,
Glycerin-1,2,3-trioctanoat,
Glycerin-1,2,3-tripelargonat,
Glycerin-1,2,3-triheptanoat (Fluka),
Mygliol 812 (bei Raumtemperatur flüssiger, gesättigter Glycerin-1,2,3-trifettsäureester mit mittelkettigen Fettsäuren, hauptsächlich Glycerin-1,2,3-trifettsäureester aus Caprylsäure und Caprinsäure),
Calciumcarbonat (gefällt, konform Ph. Eur., Fluka),
β-Tricalciumcarbonat (Eigensynthese),
Calciumsulfat-Dihydrat (Fluka, konform Ph. Eur., Fluka),
Calciumsulfat-Hemihydrat (Eigensynthese aus Calciumsulfat-Dihydrat von Fluka durch thermische Entwässerung),
6-Aminocapronsäure (Fluka),
4-(Aminomethyl)benzoesäure (Aldrich),
*trans*-4-(Aminomethyl)cyclohexan-1-carbonsäure (Tranexamsäure),
Lµtrol® micro 127 (Poly(propylenglykol-co-ethylenglycol, Aldrich), Gentamicinsulfat (Aktivitätskoeffizient 600).

### Beispiele 1 - 6:

In den Beispielen 1 - 6 wurden erfindungsgemäße Hämostyptika hergestellt, die Zusammensetzungen und Eigenschaften gemäß der nachstehenden Tabelle 1 aufwiesen.

Hierzu wurden zunächst die angegebenen Mengen an Glyerin-1,2,3-tripalmitat und Glyerin-1,2,3-trioctanoat in ein Becherglas eingewogen. Danach wurden die erhaltenen Mischungen für 30 Minuten auf 80 °C unter Rühren erwärmt, wobei eine homogene Schmelze entstand. Nach Abkühlung der Schmelze auf Raumtemperatur erfolgte die Zugabe von Lµtrol® micro 127 und gegebenenfalls Gentamicinsulfat. Die Mischungen wurden durch Kneten oder Verreiben homogenisiert, bis jeweils eine homogene, farblose Masse entstanden war.

**Tabelle 1: Zusammensetzungen und Eigenschaften der Hämostyptika gemäß den Beispielen 1 - 6.**

| **Beispiel** | **Glycerin-1,2,3-tripalmitat** | **Lµtrol® micro 127** | **Glycerin-1,2,3-trioctanoat** | **Gentamicinsulfat** | **Beurteilung** |
|---|---|---|---|---|---|
| **1** | 7,0 g | 9,5 g | 7,0 g | - | Knetbar, fest |
| **2** | 3,5 g | 9,5 g | 7,0 g | - | Knetbar, sehr weich |
| **3** | 3,5 g | 9,5 g | 6,0 g | - | Knetbar, fest |
| **4** | 7,0 g | 9,5 g | 7,0 g | 0,38 g | Knetbar, fest |
| **5** | 3,5 g | 9,5 g | 7,0 g | 0,33 g | Knetbar, sehr weich |
| **6** | 3,5 g | 9,5 g | 6,0 g | 0, 31 g | Knetbar, fest |

### Beispiele 7 - 24:

In den Beispielen 7 - 24 wurden erfindungsgemäße Hämostyptika hergestellt, die Zusammensetzungen und Eigenschaften gemäß der nachstehenden Tabelle 2 aufwiesen.

Hierzu wurden zunächst die angegebenen Mengen an den jeweiligen Glyerin-1,2,3-trifettsäureestern in ein Becherglas eingewogen. Danach wurden die erhaltenen Mischungen für 30 Minuten auf 80 °C unter Rühren erwärmt, wobei eine homogene Schmelze entstand. Nach Abkühlung der Schmelze auf Raumtemperatur erfolgte die Zugabe von Lµtrol® micro 127 und gegebenenfalls Gentamicinsulfat. Die Mischungen wurden durch Kneten oder Verreiben homogenisiert, bis jeweils eine homogene, farblose Masse entstanden war.

**Tabelle 2: Zusammensetzungen und Eigenschaften der Hämostyptika gemäß den Beispielen 7 - 24.**

| **Beispiel** | **Glycerin-1,2,3-trifettsäureester** | **Lµtrol® micro 127** | **Flüssiger Glycerin-1,2,3-trifettsäureester** | **Gentamicinsulfat** | **Beurteilung** |
|---|---|---|---|---|---|
| **7** | 3,5 g Glycerin-1,2,3-trimyristat | 9,5 g | 7,0 g Glycerin-1,2,3-trioctanoat | - | Knetbar, sehr weich |
| **8** | 3,5 g Glycerin-1,2,3-tristearat | 9,5 g | 7,0 g Glycerin-1,2,3-trioctanoat | - | Knetbar, weich |
| **9** | 3,5 g Glycerin-1,2,3-triarachinat | 9,5 g | 7,0 g Glycerin-1,2,3-trioctanoat | - | Knetbar, weich |
| **10** | 3,5 g Glycerin-1,2,3-trimyristat | 9,5 g | 7,0 g Mygliol 812 | - | Knetbar, weich |
| **11** | 3,5 g Glycerin-1,2,3-tristearat | 9,5 g | 7,0 g Mygliol 812 | - | Knetbar, weich |
| **12** | 3,5 g Glycerin-1,2,3-triarachinat | 9,5 g | 7,0 g Mygliol 812 | - | Knetbar, |
| **13** | 3,5 g Glycerin-1,2,3-tripalmitat | 9,5 g | 7,0 g Glycerin-1,2,3-triheptanoat | - | Knetbar, weich |
| **14** | 3,5 g Glycerin-1,2,3-stearat | 9,5 g | 7,0 g Glycerin-1,2,3-triheptanoat | - | Knetbar, sehr weich |
| **15** | 3,5 g Glycerin-1,2,3-tripalmitat | 9,5 g | 7,0 g glycerin-1,2,3-tripelargonat | - | Knetbar, sehr weich |
| **16** | 3,5 g Glycerin-1,2,3-trimyristat | 9,5 g | 7,0 g Glycerin-1,2,3-trioctanoat | 0,31 g | Knetbar, sehr weich |
| **17** | 3,5 g Glycerin-1,2,3-tristearat | 9,5 g | 7,0 g Glycerin-1,2,3-trioctanoat | 0,31 g | Knetbar, weich |
| **18** | 3,5 g Glycerin-1,2,3-triarachinat | 9,5 g | 7,0 g Glycerin-1,2,3-trioctanoat | 0,31 g | Knetbar, weich |
| **19** | 3,5 g Glycerin-1,2,3-trimyristat | 9,5 g | 7,0 g Mygliol 812 | 0,31 g | Knetbar, sehr weich |
| **20** | 3,5 g Glycerin-1,2,3-tristearat | 9,5 g | 7,0 g Mygliol 812 | 0,31 g | Knetbar, weich |
| **21** | 3,5 g Glycerin-1,2,3-triarachinat | 9,5 g | 7,0 g Mygliol 812 | 0,31 g | Knetbar, |
| **22** | 3,5 g Glycerin-1,2,3-tripalmitat | 9,5 g | 7,0 g Glycerin-1,2,3-triheptanoat | 0,31 g | Knetbar, weich |
| **23** | 3,5 g Glycerin-1,2,3-stearat | 9,5 g | 7,0 g Glycerin-1,2,3-triheptanoat | 0,31 g | Knetbar, sehr weich |
| **24** | 3,5 g Glycerin-1,2,3-tripalmitat | 9,5 g | 7,0 g Glycerin-1,2,3-tripelargonat | 0,31 g | Knetbar, sehr weich |

### Beispiele 25 - 58:

In den Beispielen 25 - 58 wurden erfindungsgemäße Hämostyptika hergestellt, die Zusammensetzungen und Eigenschaften gemäß den nachstehenden Tabellen 3 bis 7 aufwiesen.

Hierzu wurden zunächst die Inhaltsstoffe gemäß den Tabellen 3 bis 7 in den angegebenen Mengen in ein Becherglas eingewogen und durch Rühren miteinander vermischt. Danach wurden die erhaltenen Mischungen für 30 bis 60 Minuten auf 80 °C erwärmt. Nach Abkühlung der Schmelze auf Raumtemperatur erfolgte die Homogenisierung durch Kneten oder Verreiben, bis jeweils eine homogene farblose Masse entstanden war.

**Tabelle 3: Zusammensetzungen und Eigenschaften der Hämostyptika gemäß den Beispielen 25 - 31.**

| **Beispiel** | **Glycerin-1,2,3-trifettsäureester** | **Calciumcarbonat** | **Flüssiger Glycerin-1,2,3-fettsäureester** | **Beurteilung** |
|---|---|---|---|---|
| **25** | 7,0 g Glycerin-1,2,3-tristearat | 9,5 g | 7,0 g Glycerin-1,2,3-trioctanoat | Wachsartig, plastisch verformbar |
| **26** | 7,0 g Glycerin-1,2,3-tristearat | 9,0 g | 7,0 g Glycerin-1,2,3-trioctanoat | Wachsartig, plastisch verformbar |
| **27** | 7,0 g Glycerin-1,2,3-tristearat | 8,0 g | 7,0 g Glycerin-1,2,3-trioctanoat | Wachsartig, plastisch verformbar |
| **28** | 7,0 g Glycerin-1,2,3-trimyristat | 9,0 g | 7,0 g Glycerin-1,2,3-trioctanoat | Wachsartig, plastisch verformbar |
| **29** | 7,0 g Glycerin-1,2,3-tripalmitat | 9,0 g | 7,0 g Glycerin-1,2,3-trioctanoat | Wachsartig, plastisch verformbar |
| **30** | 7,0 g Glycerin-1,2,3-trisarachinat | 9,0 g | 7,0 g Glycerin-1,2,3-trioctanoat | Wachsartig, zäh, plastisch verformbar |
| **28** | 7,0 g Glycerin-1,2,3-trimyristat | 9,0 g | 7,0 g Mygliol 812 | Wachsartig, plastisch verformbar |
| **29** | 7,0 g Glycerin-1,2,3-tripalmitat | 9,0 g | 7,0 g Mygliol 812 | Wachsartig, plastisch verformbar |
| **30** | 7,0 g Glycerin-1,2,3-tristearat | 9,0 g | 7,0 g Mygliol 812 | Wachsartig, plastisch verformbar |
| **31** | 7,0 g Glycerin-1,2,3-trisarachinat | 9,0 g | 7,0 g Mygliol 812 | Wachsartig, zäh, plastisch verformbar |

**Tabelle 4: Zusammensetzungen und Eigenschaften der Hämostyptika gemäß den Beispielen 32 bis 40.**

| **Beispiel** | **Glycerin-1,2,3-trifettsäureester** | **Calciumsulfat-Hemihydrat** | **Calciumcarbonat** | **Flüssiger Glycerin-1,2,3-trifettsäureester** | **Beurteilung** |
|---|---|---|---|---|---|
| **32** | 6,5 g Glycerin-1,2,3-tripalmitat | 52,8 | 13,2 g | 27,5 g Glycerin-1,2,3-trioctanoat | Ohne Erwärmung plastisch verformbar, |
| **33** | 6,8 g Glycerin-1,2,3-tripalmitat | 54,6 g | 13,6 g | 25,0 g Glycerin-1,2,3-trioctanoat | Ohne Erwärmung plastisch verformbar, etwas fester als in Beispiel 32 |
| **34** | 6,5 g Glycerin-1,2,3-trimyristat | 52,8 | 13,2 g | 27,5 g Glycerin-1,2,3-trioctanoat | Ohne Erwärmung plastisch verformbar, |
| **35** | 6,5 g Glycerin-1,2,3-stearat | 52,8 | 13,2 g | 27,5 g Glycerin-1,2,3-trioctanoat | Ohne Erwärmung plastisch verformbar, |
| **36** | 6,5 g 6,5 g Glycerin-1,2,3-arachinat | 52,8 | 13,2 g | 27,5 g Glycerin-1,2,3-trioctanoat | Ohne Erwärmung plastisch verformbar, |
| **37** | 6,5 g Glycerin-1,2,3-tripalmitat | 52,8 | 13,2 g | 27,5 g Mygliol 812 | Ohne Erwärmung plastisch verformbar, |
| **38** | 6,5 g Glycerin-1,2,3-trimyristat | 52,8 | 13,2 g | 27,5 g Glycerin-1,2,3-trioctanoat | Ohne Erwärmung plastisch verformbar, |
| **39** | 6,5 g Glycerin-1,2,3-stearat | 52,8 | 13,2 g | 27,5 g Glycerin-1,2,3-trioctanoat | Ohne Erwärmung plastisch verformbar, |
| **40** | 6,5 g 6,5 g Glycerin-1,2,3-arachinat | 52,8 | 13,2 g | 27,5 g Glycerin-1,2,3-trioctanoat | Ohne Erwärmung plastisch verformbar, |

**Tabelle 5: Zusammensetzungen und Eigenschaften der Hämostyptika gemäß den Beispielen 41 und 44.**

| **Beispiel** | **Glycerin-1,2,3-tripalmitat** | **Calcium-sulfat-Dihydrat** | **Calciumcarbonat** | **Flüssiger Glycerin-1,2,3-trifettsäureester** | **Gentamicin-sulfat** | **Beurteilung** |
|---|---|---|---|---|---|---|
| **41** | 6,4 g | 51,9 g | 13,0 g | 27,1 g Glycerin-1,2,3-trioctanoat | 1,6 g | Ohne Erwärmung plastisch verformbar |
| **42** | 6,7 g | 53,7 g | 13,4 g | 24,6 g Glycerin-1,2,3-trioctanoat | 1,6 g | Ohne Erwärmung plastisch verformbar |
| **43** | 6,4 g | 51,9 g | 13,0 g | 27,1 g Mygliol 812 | 1,6 g | Ohne Erwärmung plastisch verformbar |
| **44** | 6,7 g | 53,7 g | 13,4 g | 24,6 g Mygliol 812 | 1,6 g | Ohne Erwärmung plastisch verformbar |

**Tabelle 6: Zusammensetzungen und Eigenschaften der Hämostyptika gemäß den Beispielen 45 - 50.**

| **Beispiel** | **Glycerin-1,2,3-tristearat** | **β-Tricalciumsulfat** | **Calcium carbonat** | **Glycerin-1,2,3-trioctanoat** | **ε-Aminocarbonsäure** | **Beurteilung** |
|---|---|---|---|---|---|---|
| **45** | 7,0 g | 3,5 g | 6,5 g | 7,0 g Glycerin-1,2,3-trioctanoat | 0,2 g 6-Aminocapronsäure | Ohne Erwärmung plastisch verformbar, |
| **46** | 7,0 g | 3,5 g | 6,5 g | 7,0 g Glycerin-1,2,3-trioctanoat | 0,2 g *trans*-4-(Aminomethyl)-cyclohexan-1-carbonsäure | Ohne Er-wärmung plastisch verformbar, |
| **47** | 7,0 g | - | 9,5 g | 7,0 g Glycerin-1,2,3-trioctanoat | 0,2 g 4-(Aminomethyl)benzoesäure | Ohne Erwärmung plastisch verformbar, |
| **48** | 7,0 g | 3,5 g | 6,5 g | 7,0 g Mygliol 812 | 0,2 g 6-Aminocapronsäure | Ohne Erwärmung plastisch verformbar, |
| **49** | 7,0 g | 3,5 g | 6,5 g | 7,0 g Mygliol 812 | 0,2 g *trans*-4-(Aminomethyl)-cyclohexan-1-carbonsäure | Ohne Erwärmung plastisch verformbar, |
| **50** | 7,0 g | - | 9,5 g | 7,0 g Mygliol 812 | 0,2 g 4-(Aminomethyl)benzoesäure | Ohne Erwärmung plastisch verformbar, |

**Tabelle 7: Zusammensetzungen und Eigenschaften der Hämostyptika gemäß den Beispielen 51 - 58.**

| **Rezeptur** | **Glycerin-1,2,3-trifettsäure-ester** | **Calciumcarbonat** | **Flüssiger Glycerin-1,2,3-trifettsäureester** | **Calciumchlorid** | **ε-Aminocarbonsäure** | **Beurteilung** |
|---|---|---|---|---|---|---|
| **51** | 7,0 g Glycerin-1,2,3-tristearat | 9,5 g | 7,0 g Glycerin-1,2,3-trioctanoat | 0,3 g | 0,2 g 6-Aminocapronsäure | Ohne Erwärmung plastisch verformbar, |
| **52** | 7,0 g Glycerin-1,2,3-tristearat | 9,5 g | 7,0 g Gly-cerin-1,2,3-trioctanoat | 0,3 g | 0,2 g *trans*-4-(Aminomethyl)-cyclohexan-1-carbonsäure | Ohne Er-wärmung plastisch verformbar, |
| **53** | 7,0 g Glycerin-1,2,3-tristearat | 9,5 g | 7,0 g Glycerin-1,2,3-trioctanoat | 0,2 g | 0,2 g 4-(Aminomethyl)benzoesäure | Ohne Erwärmung plastisch verformbar, |
| **54** | 7,0 g Glycerin-1,2,3-tristearat | 9,5 g | 7,0 g Mygliol 812 | 0,3 g | 0,2 g 6-Aminocapronsäure | Ohne Erwärmung plastisch verformbar, |
| **55** | 7,0 g Glycerin-1,2,3-tristearat | 9,5 g | 7,0 g Mygliol 812 | 0,3 g | 0,2 g *trans*-4-(Aminomethyl)-cyclohexan-1-carbonsäure | Ohne Erwärmung plastisch verformbar, |
| **56** | 7,0 g Glycerin-1,2,3-tristearat | 9,5 g | 7,0 g Mygliol 812 | 0,2 g | 0,2 g 4-(Aminomethyl)benzoesäure | Ohne Erwärmung plastisch verformbar, |
| **57** | 7,0 g Glycerin-1,2,3-trimyristat | 9,5 g | 7,0 g Mygliol 812 | 0,2 g | 0,2 g 4-(Aminomethyl)benzoesäure | Ohne Erwärmung plastisch verformbar, |
| **58** | 7,0 g Glycerin-1,2,3-tripalmitat | 9,5 g | 7,0 g Mygliol 812 | 0,2 g | 0,2 g 4-(Aminomethyl)benzoesäure | Ohne Erwärmung plastisch verformbar, |

Von den in den Beispielen 1 - 58 hergestellten knetbaren Massen wurde jeweils 5 g in 20 ml entionisiertes Wasser gegeben. Nach Lagerung bei Raumtemperatur für 24 Stunden wurde der pH-Wert geprüft. Es wurde in allen Fällen keine Veränderung des pH-Wertes gegenüber dem pH-Wert des entionisierten Wassers von 6,5 gemessen.

Weiterhin wurde eine Beschichtung einer Zweymueller-Hüftprothese mit den Mischungen der Beispiele 1 - 6 dadurch vorgenommen, dass die Mischungen zu Zylindern geformt wurden und anschließend diese Zylinder über die Prothesenoberflächen gestrichen wurden. Durch die Relativbewegung der strukturierten Prothesenoberfläche gegen die Zylinder wurde Material von dem Zylinder auf die Prothesenoberfläche übertragen. Es wurden je nach Anpressdruck und Wiederholung der Übertragungsvorgänge zwischen 80 und 150 mg Material der Mischungen aus den-Beispielen 1 - 6 übertragen.

## Patentansprüche

1. Plastisch verformbares, biodegradierbares Hämostyptikum enthaltend
(b) wenigstens einen zumindest teilweise in partikulärer Form vorliegenden Füllstoff, wobei die Komponente (b) aus der Gruppe ausgewählt ist, die aus Polymeren von wenigstens einem Alkylenoxid, Copolymeren von wenigstens einem Alkylenoxid und Calciumverbindungen besteht, und
(c) wenigstens einen gesättigten Fettsäureester,
**gekennzeichnet durch**
(a) wenigstens einen gesättigten Glycerin-1,2,3-trifettsäureester mit einer Schmelztemperatur von mehr als 37°C,
wobei die Calciumverbindungen Salze wenigstens einer anorganischen Säure sind und wobei die Komponente (b) eine Schmelztemperatur von mehr als 37°C und bei einer Temperatur von 25°C eine Löslichkeit von wenigstens 100 Gramm pro Liter Wasser aufweist und der wenigstens eine gesättigten Fettsäureester eine Schmelztemperatur von nicht mehr als 30°C und bei einer Temperatur von 25°C eine Löslichkeit von weniger als 50 Gramm pro Liter Wasser aufweist, wobei
die Schmelztemperatur die Temperatur bezeichnet, bei der der betreffende Stoff vom festen in den flüssigen Aggregatzustand übergeht und, wenn sich bei einem Stoff der Übergang vom festen in den flüssigen Aggregatzustand nicht bei einer bestimmten Temperatur, sondern in einem Schmelzbereich vollzieht, unter Schmelztemperatur die tiefere der beiden Grenztemperaturen dieses Schmelzbereichs verstanden wird.

2. Plastisch verformbares, biodegradierbares Hämostyptikum nach Anspruch 1, **dadurch gekennzeichnet, dass** das Hämostyptikum in Wasser bei einer Temperatur von 25°C einen pH-Wert im Bereich von 5,0 - 9,0 und vorzugsweise im Bereich von 5,5 - 8,5 aufweist.

3. Plastisch verformbares, biodegradierbares Hämostyptikum nach Anspruch 1 oder 2, **dadurch gekennzeichnet, dass** die Komponente (a) aus der Gruppe ausgewählt ist, die aus gesättigten Glycerin-1,2,3-trifettsäureestern besteht, die wenigstens einen Fettsäurerest mit 12 - 28 Kohlenstoffatomen und vorzugsweise 14 - 24 Kohlenstoffatomen aufweisen.

4. Plastisch verformbares, biodegradierbares Hämostyptikum nach einem der Ansprüche 1 - 3, **dadurch gekennzeichnet, dass** die Komponente (a) aus der Gruppe ausgewählt ist, die aus Glycerin-1,2,3-tribehensäureester, Glycerin-1,2,3-tristearinsäureester und Glycerin-1,2,3-tripalmitinsäureester besteht.

5. Plastisch verformbares, biodegradierbares Hämostyptikum nach einem der Ansprüche 1 - 4, **dadurch gekennzeichnet, dass** die Komponente (b) aus der Gruppe ausgewählt ist, die aus Poloxameren, Polyethylenglykolen und Poly(propylenglykol-co-ethylenglycol) besteht.

6. Plastisch verformbares, biodegradierbares Hämostyptikum nach einem der Ansprüche 1 - 4, **dadurch gekennzeichnet, dass** die Calciumverbindung aus der Gruppe ausgewählt ist, die aus Calciumcarbonat, Dolomit, α-Tricalciumcarbonat, β-Tricalciumcarbonat, Hydroxylapatit, Carbonatapatit, Octacalciumphosphat, amorphisiertem Calciumphosphat, Calciumsulfat-dihydrat und Calciumsulfathemihydrat besteht.

7. Plastisch verformbares, biodegradierbares Hämostyptikum nach Anspruch 7, **dadurch gekennzeichnet, dass** der gesättigte Fettsäureester aus der Gruppe ausgewählt ist, die aus (i) Estern von mehrwertigen Alkoholen mit wenigstens einer Fettsäure, (ii) Alkylfettsäureestern und (iii) Estern von Polyethern und Fettsäuren besteht.

8. Plastisch verformbares, biodegradierbares Hämostyptikum nach Anspruch 7, **dadurch gekennzeichnet, dass** der gesättigte Fettsäureester aus der Gruppe ausgewählt ist, die aus Glycerin-1,2,3-trioctylester, Mischestern von Glycerin mit Caprylsäure und Laurinsäure, Propan-1,2-dioldifettsäureestern, und Fettsäureestern von 1,3-Dihydroxy-2,2-di(hydroxymethyl)propan, Laurinsäureethylester, Myristinsäuremethylester, Myristinsäureethylester, Myristinsäureisopropylester, Palmitinsäuremethylester, Palmitinsäureethylester, Palmitinsäureisopropylester und Ethylenglykolfettsäureestern besteht.

9. Plastisch verformbares, biodegradierbares Hämostyptikum nach einem der Ansprüche 1 - 8, enthaltend 3 - 50 Gewichtsprozent der wenigstens einen Komponente (a), 10 - 80 Gewichtsprozent der wenigstens einen Komponente (b) und 10 - 50 Gewichtsprozent der wenigstens einen Komponente (c), bezogen auf das Gesamtgewicht des Hämostyptikums.

10. Plastisch verformbares, biodegradierbares Hämostyptikum nach einem der Ansprüche 1 - 9, ferner aufweisend wenigstens einen Fibrinolyseinhibitor, der aus der Gruppe der ε-Aminocarbonsäuren ausgewählt ist.

11. Plastisch verformbares, biodegradierbares Hämostyptikum nach einem der Ansprüche 1 - 10, ferner aufweisend wenigstens eine Substanz, die aus der Gruppe ausgewählt ist, die aus Antibiotika und Antiseptika besteht.

12. Verfahren zum Formen eines plastisch verformbaren, biodegradierbaren Hämostyptikums nach einem der Ansprüche 1 - 11, umfassend die Schritte:
(a) Bereitstellung eines plastisch verformbaren, biodegradierbaren Hämostyptikums nach einem der Ansprüche 1 - 11,
(b) Erwärmen des plastisch verformbaren, biodegradierbaren Hämostyptikums auf eine Temperatur im Bereich von 35 - 40°C und
(c) Formen des erwärmten plastisch verformbaren, biodegradierbaren Hämostyptikums.

13. Verfahren nach Anspruch 12, **dadurch gekennzeichnet, dass** das Erwärmen, das Formen oder das Erwärmen und das Formen durch eine Hand eines Anwenders erfolgt.

14. Medizinisches Implantat aufweisend eine Beschichtung, die ein plastisch verformbares, biodegradierbares Hämostyptikum gemäß einem der Ansprüche 1 - 11 umfasst.

## Claims

1. A malleable, biodegradable hemostatic agent containing
(b) at least one filling agent at least partially present in particulate form, wherein the component (b) is selected from the group consisting of polymers of at least one alkylene oxide, copolymers of at least one alkylene oxide and calcium compounds, and
(c) at least one saturated fatty acid ester,
**characterised by**
(d) at least one saturated glycerol-1,2,3-tri-fatty acid ester with a melting temperature of more than 37°C,
wherein the calcium compounds are salts of at least one inorganic acid and wherein the component (b) has a melting temperature of more than 37°C and a solubility at a temperature of 25°C of at least 100 grams per litre of water and the at least one saturated fatty acid ester has a melting temperature of not above 30°C, and a solubility at a temperature of 25°C of less than 50 grams per litre of water, wherein
the melting temperature identifies the temperature, at which the respective substance transitions from the solid into the liquid aggregate state and when, in the case of a substance, the transition from the solid into the liquid aggregate state does not take place at a certain temperature, but in a melting range, melting temperature is understood to be the lower of the two limit temperatures of this melting range.

2. The malleable, biodegradable hemostatic agent according to claim 1, **characterised in that** at a temperature of 25°C, the hemostatic agent has a pH value in water in the range of 5.0 - 9.0 and preferably in the range of 5.5-8.5.

3. The malleable, biodegradable hemostatic agent according to claim 1 or 2, **characterised in that** the component (a) is selected from the group consisting of saturated glycerol-1,2,3-tri-fatty acid esters, which have at least one fatty acid residue with 12 - 28 carbon atoms and preferably 14 - 24 carbon atoms.

4. The malleable, biodegradable hemostatic agent according to any one of claims 1 - 3, **characterised in that** the component (a) is selected from the group consisting of glycerol-1,2,3-tri-behenic acid ester, glycerol-1,2,3-tristearic acid ester and glycerol-1,2,3-tri-palmitic acid ester.

5. The malleable, biodegradable hemostatic agent according to any one of claims 1 - 4, **characterised in that** he component (b) is selected from the group consisting of poloxamers, polyethylene glycols and poly(propylene glycol-co-ethylene glycol).

6. The malleable, biodegradable hemostatic agent according to any one of claims 1 - 4, **characterised in that** the calcium compound is selected from the group consisting of calcium carbonate, dolomite, α-tricalcium carbonate, β-tricalcium carbonate, hydroxyapatite, carbonate apatite, octacalcium phosphate, amorphous calcium phosphate, calcium sulphate dihydrate and calcium sulphate hemihydrate.

7. The malleable, biodegradable hemostatic agent according to claim 7, **characterised in that** the saturated fatty acid ester is selected from the group consisting of (i) esters of polyvalent alcohols with at least one fatty acid, (ii) alkyl fatty acid esters and (iii) esters of polyethers and fatty acids.

8. The malleable, biodegradable hemostatic agent according to claim 7, **characterised in that** the saturated fatty acid ester is selected from the group consisting of glycerol-1,2,3-trioctylester, mixed esters of glycerol with caprylic acid and lauric acid, propane-1,2-dioldi-fatty acid esters, and fatty acid esters of 1,3-dihydroxy-2,2-di(hydroxymethyl)propane, lauric acid ethylester, myristic acid methylester, myristic acid ethylester, myristic acid isopropylester, palmitic acid methylester, palmitic acid ethylester, palmitic acid isopropylester and ethylene glycol fatty acid esters.

9. The malleable, biodegradable hemostatic agent according to any one of claims 1 - 8, containing 3 - 50 % by weight of the at least one component (a), 10 - 80 % by weight of the at least one component (b), and 10 - 50 % by weight of the at least one component (c), based on the total weight of the hemostatic agent.

10. The malleable, biodegradable hemostatic agent according to any one of claims 1 - 9, further containing at least one fibrinolysis inhibitor, which is selected from the group of the ε-aminocarbonic acids.

11. The malleable, biodegradable hemostatic agent according to any one of claims 1 - 10, further containing at least one substance, which is selected from the group consisting of antibiotics and antiseptics.

12. A method for forming a malleable, biodegradable hemostatic agent according to any one of claims 1 - 11, comprising the steps of:
(a) providing a malleable, biodegradable hemostatic agent according to any one of claims 1 - 11,
(b) heating the malleable, biodegradable hemostatic agent to a temperature in the range of 35 - 40°, and
(c) forming the heated malleable, biodegradable hemostatic agent.

13. The method according to claim 12, **characterised in that** the heating, the forming, or the heating and the forming is effected by a hand of a user.

14. A medical implant having a coating comprising a malleable, biodegradable hemostatic agent according to any one of claims 1 - 11.

## Revendications

1. Hémostatique biodégradable, déformable de manière plastique contenant
(b) au moins une charge présente au moins partiellement sous forme particulaire, dans lequel le composant (b) est sélectionné parmi le groupe qui se compose de polymères d'au moins un oxyde d'alkylène, de copolymères d'au moins un oxyde d'alkylène et de composés de calcium, et
(c) au moins un ester d'acide gras saturé,
**caractérisé par**
(a) au moins un triester de glycérol-1,2,3-acide gras saturé avec une température de fusion de plus de 37°C,
dans lequel les composés de calcium sont des sels d'au moins un acide inorganique et dans lequel le composant (b) présente une température de fusion de plus de 37°C et une solubilité d'au moins 100 grammes par litre d'eau à une température de 25°C, et l'au moins un ester d'acide gras saturé présente une température de fusion de pas plus de 30°C et une solubilité de moins de 50 grammes par litre d'eau à une température de 25°C, dans lequel la température de fusion désigne la température à laquelle la substance concernée passe de l'état solide à l'état liquide et, lorsque la transition de l'état solide à l'état liquide pour une substance n'a pas lieu à une température déterminée, mais dans une plage de fusion, il est compris par température de fusion la plus basse des deux températures limites de cette plage de fusion.

2. Hémostatique biodégradable, déformable de manière plastique selon la revendication 1, **caractérisé en ce que** l'hémostatique présente une valeur de pH dans la plage de 5,0 à 9,0 et de préférence dans la plage de 5,5 à 8,5 dans de l'eau à une température de 25°C.

3. Hémostatique biodégradable, déformable de manière plastique selon la revendication 1 ou 2, **caractérisé en ce que** le composant (a) est sélectionné parmi le groupe qui se compose de triesters de glycérol-1,2,3-acide gras saturés qui présentent au moins un résidu d'acide gras avec 12 à 28 atomes de carbone et de préférence 14 à 24 atomes de carbone.

4. Hémostatique biodégradable, déformable de manière plastique selon une des revendications 1 à 3, **caractérisé en ce que** le composant (a) est sélectionné parmi le groupe qui se compose du triester de glycérol-1,2,3-acide béhénique, du triester de glycérol-1,2,3-acide stéarique et du triester de glycérol-1,2,3-acide palmitique.

5. Hémostatique biodégradable, déformable de manière plastique selon une des revendications 1 à 4, **caractérisé en ce que** le composant (b) est sélectionné parmi le groupe qui se compose de poloxamères, polyéthylèneglycols et poly(propylèneglycol-co-éthylèneglycol).

6. Hémostatique biodégradable, déformable de manière plastique selon une des revendications 1 à 4, **caractérisé en ce que** le composé de calcium est sélectionné parmi le groupe qui se compose du carbonate de calcium, de la dolomie, de l'α-tricarbonate de calcium, du β-tricarbonate de calcium, de l'hydroxyapatite, de la carbonate-apatite, de l'octaphosphate de calcium, du phosphate de calcium amorphisé, du dihydrate de sulfate de calcium et de l'hémihydrate de sulfate de calcium.

7. Hémostatique biodégradable, déformable de manière plastique selon la revendication 7, **caractérisé en ce que** l'ester d'acide gras saturé est sélectionné parmi le groupe qui se compose de (i) esters d'alcools polyvalents avec au moins un acide gras, (ii) esters alkyliques d'acide gras et (iii) esters de polyéthers et d'acides gras.

8. Hémostatique biodégradable, déformable de manière plastique selon la revendication 7, **caractérisé en ce que** l'ester d'acide gras saturé est sélectionné parmi le groupe qui se compose du triester de glycérol-1,2,3-octyle, d'esters mixtes de glycérol avec de l'acide caprylique et de l'acide laurique, de diesters d'acide gras de propane-1,2-diol et d'esters d'acide gras de 1,3-dihydroxy-2,2-di(hydroxyméthyl)propane, de l'ester éthylique d'acide laurique, de l'ester méthylique d'acide myristique, de l'ester éthylique d'acide myristique, de l'ester isopropylique d'acide myristique, de l'ester méthylique d'acide palmitique, de l'ester éthylique d'acide palmitique, de l'ester isopropylique d'acide palmitique et d'esters d'acide gras d'éthylèneglycol.

9. Hémostatique biodégradable, déformable de manière plastique selon une des revendications 1 à 8, contenant 3 à 50 pourcent en poids de l'au moins un composant (a), 10 à 80 pourcent en poids de l'au moins un composant (b) et 10 à 50 pourcent en poids de l'au moins un composant (c) par rapport au poids total de l'hémostatique.

10. Hémostatique biodégradable, déformable de manière plastique selon une des revendications 1 à 9, présentant en outre au moins un inhibiteur de fibrinolyse qui est sélectionné parmi le groupe des acides ε-aminocarboxyliques.

11. Hémostatique biodégradable, déformable de manière plastique selon une des revendications 1 à 10, présentant en outre au moins une substance qui est sélectionnée parmi le groupe qui se compose d'antibiotiques et d'antiseptiques.

12. Procédé de moulage d'un hémostatique biodégradable, déformable de manière plastique selon une des revendications 1 à 11, comprenant les étapes de :
(a) mise à disposition d'un hémostatique biodégradable, déformable de manière plastique selon une des revendications 1 à 11,
(b) chauffage de l'hémostatique biodégradable, déformable de manière plastique à une température dans la plage de 35 à 40°C et
(c) moulage de l'hémostatique biodégradable, déformable de manière plastique chauffé.

13. Procédé selon la revendication 12, **caractérisé en ce que** le chauffage, le moulage ou le chauffage et le moulage s'effectue(nt) par une main d'un utilisateur.

14. Implant médical présentant un revêtement qui comprend un hémostatique biodégradable, déformable de manière plastique selon une des revendications 1 à 11.
